# EUROPEAN PATENT APPLICATION

(11) **EP 0 917 866 A1**
(43) Date of publication of application: **26.05.1999**
(21) Application number: 97119753.8
(22) Date of filing: 12.11.1997
(51) Int. Cl.: A61F 13/56

(54) **Long sanitary napkins having stepped panty fastening adhesive**

(71) Applicant: THE PROCTER & GAMBLE COMPANY, Cincinnati, Ohio 45202 (US)
(72) Inventor: Häsler, Peter Ludwig, 65510 Idstein (DE); Kreutz, Karen Ann, 61381 Friedrichsdorf (DE); Lawton, Rachel Ann, 60929 Frankfurt (DE); Linzmaier, Ulrike Barbara, 69493 Hirschberg/Grosssachsen (DE); Steigert, Christian Klaus, 67434 Neustadt (DE)
(74) Representative: Hirsch, Uwe Thomas

(57) **Abstract**

The present invention relates to absorbent articles such as sanitary napkins, catamenials or adult incontinence inserts which are used in combination with undergarments. The absorbent articles according to the present invention have a minimum length of 270 mm, i.e. they are relatively long absorbent articles, and they are provided with a fastening adhesive to attach them to the undergarment during use. In particular the present invention relates to such absorbent articles which are not symmetrical in longitudinal direction, i.e. they extend when placed on a wearer more to the front or more to the rear considering the perineal area of a female wearer of such articles. The fastening adhesive is also provided with an asymmetry in respect to the longitudinal direction.

## Description

### Field of the invention

The present invention relates to absorbent articles such as sanitary napkins, catamenials or adult incontinence inserts which are used in combination with undergarments. The absorbent articles according to the present invention have a minimum length of 255 mm, i.e. they are relatively long absorbent articles, and they are provided with a fastening adhesive to attach them to the undergarment during use. In particular the present invention relates to such absorbent articles which are not symmetrical in longitudinal direction, i.e. they extend when placed on a wearer more to the front or more to the rear considering the perineal area of a female wearer of such articles. The fastening adhesive is provided with an asymmetry in respect to the longitudinal direction.

### Background of the invention

Fastening adhesive for sanitary napkins are well-known in the art. Usually they cover part of the garment facing surface of sanitary napkins. The adhesive allows improved fixation of the napkins to the undergarment when applying the product and during use.

The dimensions of such panty fastening adhesives are influenced by several factors. One aspect is that they should cover as large an area as possible in order to provide firm attachment of the napkin to the undergarment. On the other hand the adhesive must not extend to areas which could possibly attach to the skin of the wearer since that would cause an unacceptable discomfort when using the product. Therefore, when considering the crotch portion of an undergarment typically products having been provided with an adhesive along the longitudinal length of the sanitary napkin such that the adhesive cannot extend beyond the smallest crotch width of the undergarment minus a certain safety margin to allow variation in placement of the napkin in the undergarment depending on user accuracy.

The other aspect when deciding on the panty fastening adhesive dimensions is how to provide the panty fastening adhesive onto the garment facing surface of a sanitary napkin in a mass production process. Many processes have been considered in this respect. EP-A-745 386 discloses printing of panty fastening adhesives in order to allow any shape of panty fastening adhesives to be provided. However, it is still most common to provide panty fastening adhesives by continuous coating or continuous spraying. The panty fastening adhesive is applied while the sanitary napkin moves on a band or belt in longitudinal direction. This automatically results in straight adhesive dimensions, which, when combined with an on-off switching of the adhesive application system, creates a rectangular stripe, or a series of rectangular stripes, on the garment facing surface of a sanitary napkin. By default these stripes cannot reach into a region of the sanitary napkin which extends beyond the smallest transverse dimension, i.e. width, of a sanitary napkin. In practical terms the adhesive needs to be even smaller in order to not extend to the peripheral edges of the sanitary napkin in the crotch region since this is also known to cause problems in comfort for the user, compare for example WO 92/04000.

Hence it has become usual to provide sanitary napkins with a single or multitude of rectangular longitudinal stripes of panty fastening adhesive on their garment facing surface. This is acceptable for relatively short sanitary napkins of less than 255 mm, typically of less 240 mm. However, sanitary napkins extending along a larger length also are exposed to more variation in the forces they are exerted to (e.g. they extend from the crotch region beyond the public mons or beyond the anus into the buttocks region) and hence would benefit from better fixation to the undergarment, especially at the ends.

There is one further reason for not exceeding a maximum width in the crotch area of a panty fastening adhesive (even if a sanitary napkin would allow to provide an adhesive area wider than this certain width, which reason is that the crotch width of undergarments is limited and hence providing adhesive beyond this crotch width is not useful but rather counter productive since it may expose adhesive to the skin of the wearer). This, however, is not the case outside the crotch region of the undergarment where the undergarment flares out into the front portion and into the rear portion covering the buttocks.

Based on the above an objective of the present invention is to provide long sanitary napkins, and in particular those which are asymmetrically shaped, i.e. having a longer front or rear portion with a better attachment to the undergarment without violating the fundamental conditions of comfort (i.e. not extending beyond the crotch width of the undergarment with the adhesive) and manufacturing simplicity, for example by use of adhesive coating or spraying with no more variation than an on/off-switching mechanism.

### Summary of the invention

The present invention relates to a sanitary napkin for use in an undergarment having a garment facing surface and having a longitudinal centerline and a transverse centerline perpendicular to the longitudinal centerline which define a longitudinal direction and a transverse direction. The sanitary napkin comprises 3 regions, a first region in the center which has a length of at least 40 mm to cover the area on a wearer between the perineum and the most forward point of the labia majora during use. A second region extends in longitudinal direction on one side of the first region and a third region which extends on the opposite side of the first region. The second region is longer than the third region such that the sanitary napkin is worn in an asymmetric fashion. The length in longitudinal direction of the sanitary napkin is at least 255 mm, preferably 270 mm and most preferably 300 mm or more.

The garment facing surface of the sanitary napkin comprises an adhesive for attachment of the sanitary napkin to an undergarment. The adhesive extends from the second region through the first region into the third region and has a width dimension in transverse direction at least in one point of the second region which is larger than the width in either the first or the third region. More particularly the adhesive in the first and third region is provided in a rectangular pattern relative to the centerlines and has a width in transverse direction between 35 and 60 mm while the adhesive in at least one point of the second region has a width in transverse direction of more than the width in the first and second region, preferably in the range 45 to 100 mm. In an even more preferable embodiment the adhesive in the second region is also rectangular relative to the centerlines.

In another embodiment according to the present invention the sanitary napkin also comprises wings which are well-known in the art. The wings extend a length of at least 80 % of the central first region of the sanitary napkin and preferably extend no more than 20 % of the length of the third region into the third region while even more preferable the wings do not extend more than 50 % of the length of the second region into the second region.

### Brief description of the drawings

Figure 1 shows a plan view of the garment facing surface of a sanitary napkin of the prior art.
Figure 2 shows a preferred embodiment of a sanitary napkin according to the present invention.
Figure 3 shows an alternative embodiment of the present invention with different adhesive dimensions on one side of the longitudinal direction as a first alternative and on the other side of the longitudinal centerline as a second alternative.
Figure 4 shows another alternative embodiment according to the present invention.

### Detailed description of the invention

According to the present invention an absorbent article for use in combination with an undergarment has an adhesive for attachment of the article to the undergarment. The design of the adhesive is particularly adjusted to the dimensions of the article and is asymmetric in longitudinal direction and reaches in use into a region in the undergarment distant from the crotch center.

In particular, sanitary napkins, catamenials and panty liners whether used for incontinence discharges or menstrual or other discharges are considered to be susceptible to the present invention. A typical absorbent article susceptible to the present invention is a sanitary napkin liner having an absorbent structure overlaid on the body facing side by a liquid permeable topsheet and overlaid on the garment facing side by a liquid impermeable backsheet. Optionally, the article may comprise wings or flaps laterally extending from the central portion of the article away from the longitudinal axis and folded around the crotch portion of an undergarment during use. Details on the articles contemplated in combination with the present invention can be found in many patent disclosures and include those absorbent articles commercially available.

The sanitary napkin comprises an adhesive area on its garment facing surface which adhesive is protected by a cover means which cover means is released prior to use of the article. The adhesive area need not be fully covered by adhesive but may for example be provided by homogeniously distributed adhesive filaments leaving small areas uncovered between the filaments. Alternatively the "adhesive area" according to the present invention may be provided by sub portions of adhesive and is then defined as the area formed by the shortest possible line encircling all sub portions, however excluding those in the wings.

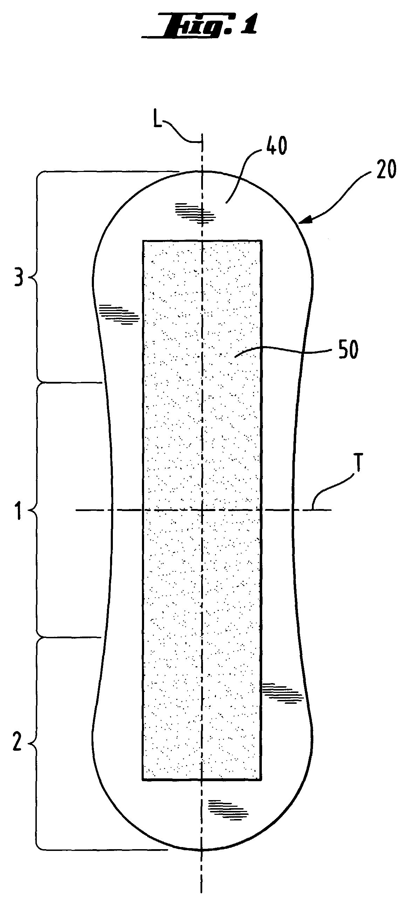

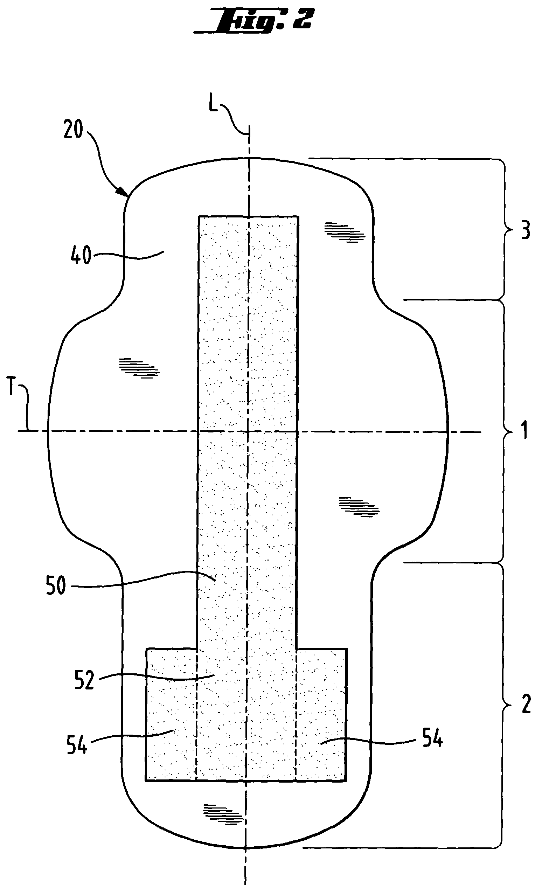

As can be seen in the prior art sanitary napkin (20) of figure 1 the garment facing side (40) which typically is provided by a liquid impermeable backsheet of such a sanitary napkin, comprises an adhesive (50). In general sanitary napkins have a longitudinal centerline (L) and a transverse centerline (T). In the prior art sanitary napkin of Figure 1 the transverse centerline (T) is located in the middle of the sanitary napkin, i.e. it separates the sanitary napkin into two halves which are approximately of equal length in longitudinal direction. The shown sanitary napkin is also symmetrical in longitudinal direction to the transverse centerline (T). In contrast a sanitary napkin according to the present invention such as the sanitary napkins shown in figure 2 , 3 or 4 has a transverse centerline (T) which separates a napkin into two halves which are substantially not identical in length.

The transverse centerline (T) is located in a central or first region of a sanitary napkin. This first region has an extension in longitudinal direction sufficient and intended to cover the area on a wearer between the perineum and the most forward point of the labia majora. Based on medical studies this varies between about 40 mm and 80 mm on average women depending on sexual activity, child birth and other factors. Hence the minimum length of the first region is 40 mm but would preferably be at least 60 mm, more preferably at least 80 mm and most preferably at least 112 mm. The transverse centerline separates the first region into two halves in longitudinal direction which are substantially of equal length. This is obviously the case for conventional sanitary napkins of the prior art or for sanitary napkins of the present invention.

Sanitary napkins having a first region (1) according to the above definition also have a second (2) and a third (3) region outside the first region which are extending from the first region (1) to the longitudinal ends of the sanitary napkin. As can be seen from the figures 2, 3 and 4 sanitary napkins according to the present invention have a second region (2) which is longer than the third region (3).

A panty fastening adhesive (50) in prior art sanitary napkins which are longitudinally symmetrical as shown in figure 1 will naturally extend an equal distance from said first region (1) into said second (2) and said third region (3). In contrast sanitary napkins according to the present invention have an undergarment adhesive attachment (50) which preferably extends in longitudinal direction further from the first region (1) into the second region (2) then it extends from the first region into the third region (3), as shown in the figures 2, 3 and 4.

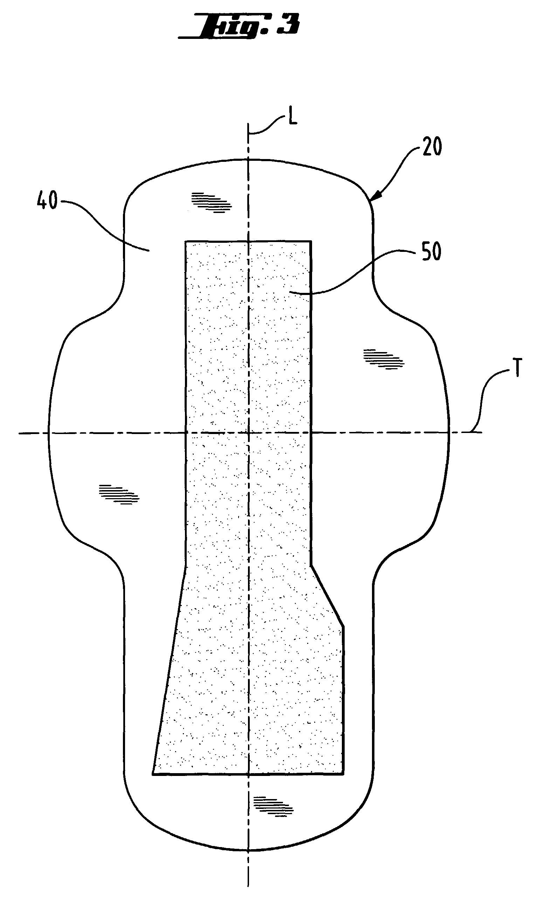

According to the present invention the width in transverse direction of the adhesive in the second region (2) is wider in at least one point than the width of the adhesive in the first (1) and in the third region (3). Figure 2 shows a particularly preferred shape of adhesive in which the adhesive is provided in a rectangular pattern, which is easy to provide when continuously manufacturing sanitary napkins in longitudinal direction. Two alternative shapes satisfying the width requirement according to the present invention are shown in figure 3 on the right and left side of the longitudinal axis. Both of the designs of the adhesive (50) shown in figure 3 also comprise non-rectangular portions. Another preferred embodiment is shown in figure (4) in which the wider portion of the adhesive (50) in the second region (2) is provided in a semi-oval shape.

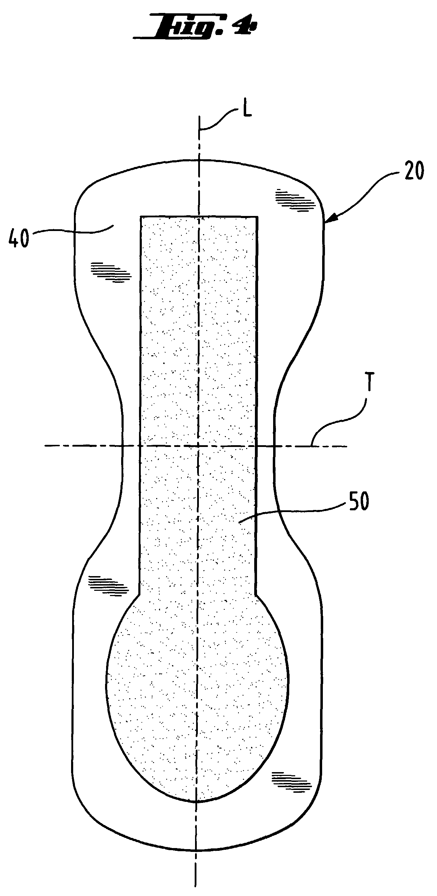

It is also possible that the adhesive (50) in the wider portion of the adhesive in the second region (2) of sanitary napkins according to the present invention may comprise a different quantity of adhesive than the adhesive in the first or second region. For example the pattern shown in figure 2 can be provided by coating an adhesive in longitudinal direction through three coating means (e.g. slot coaters or spray nozzles). One coating means providing a central rectangular area (54) extending from the third (3) through the first (1) to the second (2) region (indicated by dashes in the second region (2)) and being similar to the adhesive shown in figure 1 for prior art sanitary napkins. In addition the areas of adhesive (54) extending beyond the rectangular portion (52) in the second region (2) of the sanitary napkin are provided by two additional coating means which may provide the same amount of adhesive per area or may provide a different amount of adhesive per area than the central coating means. In principle it would also be possible to provide different adhesives through the different coating means. In this context also stripes of adhesive instead of a completely coated surface could be provided. Then the adhesive area shown in Figure 2 is the area inside the shortest possible line encircling all sup portions, excluding adhesive on the wings (not shown in Figure 2). The same applies to Figures 3 and 4.

In an alternative way of providing the adhesive to the garment facing surface (40) of a sanitary napkin according to the present invention it would also be possible to provide the whole area in which the adhesive is wider in the second region (2) by a separate coating means while the adhesive in the first and third region of the sanitary napkin is provided by conventional adhesive coating means such as those used for prior art sanitary napkins according to figure 1.

As noted above sanitary napkins according to the present invention are preferably provided with flaps or wings extending beyond the undergarment crotch width and being folded around the undergarment crotch so as protect the undergarment side edges. Sanitary napkins with wings are shown in the preferred embodiments of figures 2 and 3 while wings are not mandatory but only preferred in the context of the present invention since they provide additional fixation of sanitary napkins, particularly very long sanitary napkins.

Preferably as indicated in the background discussion above the width of the adhesive in the first region is such that it extends close to the side edges of the napkin as shown in figure 4.

The adhesive for fastening the article in an undergarment can be any adhesive useful for the desired application. Usually it is a pressure sensitive adhesive which can be applied in any way usual in the art. In particularly, the adhesive application can be by use of contact application means, such as printing or scraping or slot coating, or by non contact application, such as curtain coating, spraying or spiraling. The adhesive can be coated as hot melt or from a cold solution, either to the garment facing surface of the article or to the cover means which then carries it to the article.

The adhesive needs to be adapted to the desired application, e.g. it needs to support breathability if it is used in a breathable sanitary napkin. The adhesion force of the adhesive to the undergarment facing surface of the article needs to be larger or equal to that adhesion force which it has to the cover means. Also it is desirable to have an adhesive which does not separate during use from the article and leaves adhesive traces in the undergarment when separating the article from the undergarment.

It will be appreciated by those skilled in the art that the above explanations and embodiments are disclosed for the purpose of illustration and enablement but that the present invention is defined by the limits given in the claims.

## Claims

1. A sanitary napkin (20) for use in an undergarment, said sanitary napkin (20) having a garment facing surface, said sanitary napkin (20) having a longitudinal centerline (L) and a transverse centerline (T) perpendicular to said longitudinal centerline (L), said sanitary napkin (20) comprising three regions (1, 2, 3) along said longitudinal centerline (L),
a first region (1) which includes said transverse centerline (T) and which has a length of at least 40 mm to cover in use the area on a wearer along said longitudinal direction between the perineum and the most forward point of the labia majora,
a second region (2) which extends along said longitudinal direction on one side of said first region (1), and
a third region (3) which extends along said longitudinal direction on the opposite side of said first region,
said second region (2) being longer than said third region (3),
said sanitary napkin (20) having a length of at least 255 mm, in longitudinal direction,
said garment facing side (40) of said sanitary napkin (20) comprising an adhesive (50) for attachment to said undergarment,
said sanitary napkin (20) being characterised in that
said adhesive (50) extends from said second region (2) through said first region (1) into said third region (3) and in that the adhesive (50) width in transverse direction at least at one point of said second region (2) is larger than the adhesive width in transverse direction at one point of said first region (1) and of said third region (3).

2. A sanitary napkin (20) according to claim 1 characterised in that said adhesive (50) is said first region (1) and third region (3) is provided in a rectangular pattern relative to said centerlines (L, T) and has a width in transverse direction in the range of 35 mm to 60 mm in said first region (1) and said third region (3) and said adhesive (50) has a width in transverse direction in the range of 45 mm to 110 mm at least at one point in said second region (2).

3. A sanitary napkin (20) according to any of the preceding claims characterised in that said adhesive (50) in said second region (2) is provided in a rectangular pattern relative to said centerlines (L, T).

4. A sanitary napkin (20) according to any of the preceding claims characterised in that said sanitary napkin (20) has a length of at least 270 mm, preferably at least 300 mm.

5. A sanitary napkin (20) according to any of the preceding claims characterised in that said sanitary napkin (20) further comprises wings (25) having an extension in longitudinal direction of at least 80 % of the length of said first portion (1) and extending at least within said first region (1), preferably not extending more than 20 % of the length of said third portion (3) into said third portion (3) and more preferably not extending more than 15 % of the length of said second portion (2) into said second portion (2).
